(19) Europäisches Patentamt
European Patent Office
Office européen des brevets

(11) Numéro de publication : **0 021 952**
**B1**

(12) **FASCICULE DE BREVET EUROPÉEN**

(45) Date de publication du fascicule du brevet : **03.11.82**

(21) Numéro de dépôt : **80400846.4**

(22) Date de dépôt : **11.06.80**

(51) Int. Cl.³ : **C 07 C 33/14**, C 07 C 29/48, C 07 C 29/60// C07C69/007, C07C67/08, C11B9/00, C07C47/46

(54) **Procédé pour la préparation de l'alcool périllique et de ses dérivés.**

(30) Priorité : **19.06.79 FR 7915676**

(43) Date de publication de la demande :
**07.01.81 (Bulletin 81/01)**

(45) Mention de la délivrance du brevet :
**03.11.82 Bulletin 82/44**

(84) Etats contractants désignés :
**CH DE GB IT LI NL**

(56) Documents cités :
**FR A 1 183 849**
**FR A 2 232 533**

**BULLETIN DE LA SOCIETE CHIMIQUE DE FRANCE, vol. 7, juillet 1958, Paris, FR J. PHILLBERT et al. « Une synthèse de l'alcool périllique », pages 1 174-1 178**

**CHEMICAL ABSTRACTS, vol. 69, 15 juillet 1968, no 3, page 1 010, réf. 10547x Columbus, Ohio, USA SATO TAKESHI : « A product of oxidation of beta-pinene with lead tetra, acetate »**

**CHEMICAL ABSTRACTS, vol. 64, 28 mars 1966, no 7, page 9 776, Columbus Ohio, USA**

**CHEMICAL ABSTRACTS, vol. 85, 2 août 1976, no 5, page 418, ref. 33220e, Columbus, Ohio, USA**

(73) Titulaire : **SYNAROME - H. FRAYSSE ET CIE**
**Zone Industrielle de Beaulieu Rue Charles Tellier**
**F-28000 Chartres (FR)**

(72) Inventeur : **Fetizon, Marcel**
**104 rue de Damiette**
**F-91190 Gif sur Yvette (FR)**
Inventeur : **Ecoto, Jules Ebelle**
**5 Résidence Clos du Pileu**
**F-91120 Palaiseau (FR)**
Inventeur : **Lazare, Sylvain**
**9 Avenue de Chevincourt Cressely**
**F-78470 Saint Remy les Chevreuse (FR)**

(74) Mandataire : **Bouju, André**
**38 Avenue de la Grande Armée**
**F-75017 Paris (FR)**

EP 0 021 952 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé pour la préparation de l'alcool périllique et de ses dérivés

La présente invention concerne un procédé pour la préparation de l'alcool périllique ou hydroxy-7 paramenthadiène 1-8 et d'un dérivé de cet alcool : le dihydroxy-7,8 paramenthène-1.

L'alcool périllique, dont la formule est développée ci-après :

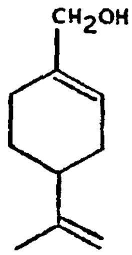

est remarquable par son odeur agréable. Ce composé est couramment utilisé en tant que parfum, en cosmétique, parfumerie et savonnerie.

On connaît différents procédés pour préparer l'alcool périllique.

Selon un premier procédé, on oxyde de l'alpha ou du bêta-pinène par un tétracarboxylate de plomb (voir brevet britannique n° 1 094 875).

Selon un second procédé connu, on oxyde de l'alpha ou bêta-pinène (extrait du pin) par le péroxyde de benzoyle en présence de sels de cuivre (voir demande de brevet allemand n° 2 162 882).

Ces deux procédés ont l'inconvénient de présenter de mauvais rendements, et par suite de conduire à un mélange de composés dans lequel il est difficile d'extraire l'alcool périllique pur.

Dans un autre procédé connu, on utilise comme produit de départ du limonène. Ce procédé est onéreux, car le limonène est lui-même un produit cher.

Le but de la présente invention est de fournir un procédé pour préparer l'alcool périllique présentant, entre autres avantages, d'être économique, de mise en œuvre aisée et permettant d'obtenir d'excellents rendements.

Suivant l'invention, ce procédé est caractérisé en ce qu'on fait réagir de l'époxyde de bêta-pinène avec une suspension aqueuse de sel mercurique, on extrait du milieu réactionnel la phase organique obtenue, et on traite cette phase organique par un acide minéral.

La réaction est rapide (environ 10 minutes), et ne nécessite aucun chauffage.

La réaction s'écrit comme suit :

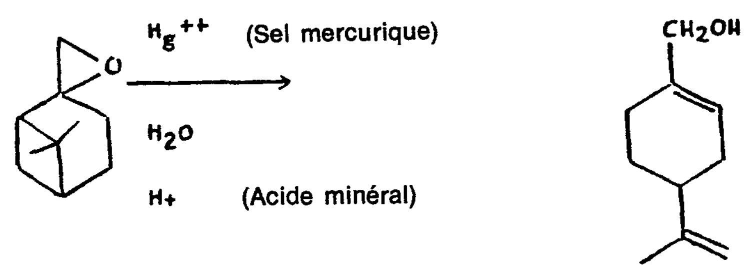


bêta-pinène — alcool périllique

Le sel mercurique utilisé est, de préférence, du sulfate mercurique ($Hg\ SO_4$) ou du chlorate mercurique ($(ClO_4)_2\ Hg$).

Pour obtenir un rendement proche de 100 %, il est avantageux d'utiliser une quantité de sel mercurique en excès par rapport à la quantité stoechiométrique théoriquement nécessaire.

Egalement, pour favoriser la réaction, il est avantageux que cette dernière soit effectuée en présence d'un solvant organique miscible à l'eau, tel que le tétrahydrofuranne. On obtient ainsi un milieu réactionnel parfaitement homogène.

On donne ci-après un exemple non limitatif de mode opératoire pour la préparation de l'alcool périllique.

## Exemple I

A une suspension de 2,96 g de $HgSO_4$ dans 10 $cm^3$ de $H_2O$ et 10 $cm^3$ de tétrahydrofuranne, on ajoute

1,5 g d'époxyde de bêta-pinène. Après 10 minutes, la réaction est terminée. On contrôle la fin de la réaction par chromatographie en couche mince. On extrait la phase organique à l'éther, lave à l'acide sulfurique dilué (ou avec tout autre acide minéral dilué), puis avec une solution aqueuse de $Na_2CO_3$, sèche, on chasse et distille le solvant. On sépare par chromatographie liquide sous haute pression pour obtenir un échantillon analytique.

Par évaporation de la phase aqueuse, on récupère le sulfate mercurique.

On obtient ainsi de l'alcool périllique, avec un rendement supérieur à 98 %.

L'époxyde de bêta-pinène utilisé comme produit de départ dans cette réaction s'obtient facilement en oxydant de façon sélective le bêta-pinène.

L'époxyde de bêta-pinène peut être lévogyre, destrogyre ou racémique.

L'expérience a montré ce résultat surprenant, qu'en modifiant légèrement le mode opératoire du procédé conforme à l'invention, il était possible d'obtenir un dérivé parfaitement stable de l'alcool périllique constitué par le dihydroxy-7,8 paramenthène-1 de formule :

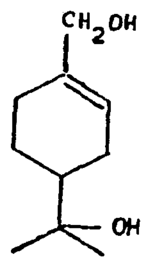

Conformément à l'invention, pour préparer du dihydroxy-7,8 paramenthène-1, on procède comme pour la préparation de l'alcool périllique, en omettant de traiter la phase organique par un acide minéral.

Le dihydroxy-7,8 paramenthène-1 présente une odeur « fraîche, légèrement verte, aromatique » très agréable qui justifie son application dans les compositions parfumantes destinées à la parfumerie, aux produits de cosmétique, aux savons et détergents, aux déodorants d'ambiance et aux produits industriels.

On donne ci-après un exemple de mode opératoire pour la préparation du dihydroxy-7,8 paramenthène-1.

Exemple II

En opérant comme dans l'exemple I, en utilisant 6 g de $Hg(ClO_4)_2$ et 2 g d'époxyde de β-pinène (ou 2,96 g de $HgSO_4$ et 1,5 g d'époxyde de β-pinène), et en omettant le lavage final à l'acide minéral, on obtient avec un rendement compris entre 98 et 100 % du dihydroxy-7,8 paramenthène-1 pratiquement pur, qui peut être utilisé tel quel.

Par chromatographie sous haute pression, on obtient un échantillon analytique.

L'analyse montre que ce composé a pour formule brute $C_{10}H_{18}O_2$.

La structure de ce composé est établie par R.M.N. (Résonance magnétique nucléaire) par $12_C$ et $1_H$.

Le monoacétate du dihydroxy-7,8 paramenthène-1 de formule :

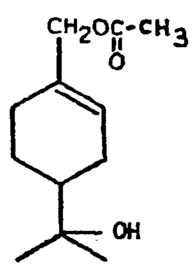

est préparé par estérification classique par l'acide acétique.

Ce composé présente également une odeur boisée encens, balsamique, caractéristique très agréable.

Les esters supérieurs, monopropionate, monobutylate, etc... peuvent de même être préparés par estérification classique.

L'aldéhyde de formule :

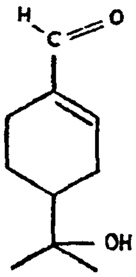

peut également être préparée selon la méthode classique de déshydratation des alcools primaires.

On donne ci-après quelques exemples non limitatifs de compositions renfermant un dérivé conforme à l'invention :

Exemple I

| | |
|---|---|
| Acétate de linalyle | 17 (parties en poids) |
| Acétate de terpényle | 15 (parties en poids) |
| Acétate de géranyle | 5 (parties en poids) |
| Coumarine | 7 (parties en poids) |
| Romarin | 10 (parties en poids) |
| Terpinéol | 7 (parties en poids) |
| Nopol | 20 (parties en poids) |
| Ethyl amylcétone | 2 (parties en poids) |
| Dihydroxy-7,8 paramenthène-1 | 17 (parties en poids) |

Exemple II

| | |
|---|---|
| Aldéhyde hexyl cinnamique | 10 (parties en poids) |
| Géranio | 10 (parties en poids) |
| Diméthyl benzyl carbinol | 10 (parties en poids) |
| Acétate de terpényle | 15 (parties en poids) |
| Composition jasmin | 30 (parties en poids) |
| Composition oranger | 2 (parties en poids) |
| Héliotropine | 1 (partie en poids) |
| Essence de styrax | 2 (parties en poids) |
| Linalol | 5 (parties en poids) |
| Diméthyl acétal du citral | 3 (parties en poids) |
| Composition rose | 6 (parties en poids) |
| Dihydroxy-7,8 paramenthène-1 | 6 (parties en poids) |

Exemple III

| | |
|---|---|
| Coumarine | 10 (parties en poids) |
| Bergamote non sensibilisante | 10 (parties en poids) |
| Alcool phényl éthylique | 4 (parties en poids) |
| Méthylionone | 15 (parties en poids) |
| Vétiver | 10 (parties en poids) |
| Acétate de cédryle | 7 (parties en poids) |
| Acétate de vétiveryle | 5 (parties en poids) |
| Composition jasmin | 6 (parties en poids) |
| Santal | 10 (parties en poids) |
| Aldéhyde C.II I/10 | 3 (parties en poids) |
| Aldéhyde C.12 I/10 | 1 (partie en poids) |
| Géraniol | 5 (parties en poids) |
| Citronéllol | 3 (parties en poids) |
| Acétate de dihydroxy-7,8 paramenthène-1 | 11 (parties en poids) |

**Revendications**

1. Procédé pour la préparation de l'alcool périllique, caractérisé en ce qu'on fait réagir de l'époxyde

de bêta-pinène avec une suspension aqueuse de sel mercurique, on extrait du milieu réactionnel la phase organique obtenue et on traite cette phase organique par un acide minéral.

2. Procédé conforme à la revendication 1, caractérisé en ce que le sel mercurique utilisé est choisi dans le groupe comprenant le sulfate mercurique et le chlorate mercurique.

3. Procédé conforme à l'une quelconque des revendications 1 ou 2, caractérisé en ce qu'on utilise une quantité de sel mercurique en excès par rapport à la quantité stoechiométrique.

4. Procédé conforme à l'une quelconque des revendications 1 à 3, caractérisé en ce que la réaction est effectuée en présence d'un solvant organique miscible à l'eau.

5. Procédé conforme à la revendication 4, caractérisé en ce que le solvant utilisé est du tétrahydrofuranne.

6. Procédé pour la préparation du dihydroxy-7,8 paramenthène-1, caractérisé en ce qu'on procède selon l'une quelconque des revendications 1 à 5, en omettant de traiter la phase organique par un acide minéral.

**Claims**

1. Method for the preparation of perillic alcohol, characterized in that beta-pinene epoxide is reacted with an aqueous suspension of mercuric salt, the obtained organic fraction is extracted from the reaction medium and said organic fraction is treated with an inorganic acid.

2. Method according to claim 1, characterized in that said mercuric salt is choosen among the group comprising mercuric sulphate and mercuric chlorate.

3. Method according to claim 1 or 2, characterized in that said reaction is performed with a stoechiometric excess of the mercuric salt.

4. Method according to any one of claims 1 to 3, characterized in that said reaction is performed in the presence of a water-miscible organic solvent.

5. Method according to claim 4, characterized in that said solvent is tetrahydrofuranne.

6. Method for the preparation of 7,8-dihydroxy 1-paramenthene, characterized in that the method according to any one of claims 1 to 5 is followed except that the treatment of said organic fraction with an inorganic acid is omitted.

**Ansprüche**

1. Verfahren zur Herstellung von Perillaalkohol, dadurch gekennzeichnet, daß man das Epoxyd von beta-Pinen mit einer wäßrigen Quecksilber-II-salzsuspension reagieren läßt, die erhaltene organische Phase aus dem Reaktionsmilieu extrahiert und diese organische Phase mit einer Mineralsäure behandelt.

2. Verfahren nach Anspruch 1, dadurch gekennzeichnet, daß das verwendete Quecksilber-II-salz Mercurisulfat oder Mercurichlorat ist.

3. Verfahren nach einem der Ansprüche 1 oder 2, dadurch gekennzeichnet, daß man eine Quecksilber-II-salzmenge verwendet, die sich im Überschuß gegenüber der stöchiometrischen Menge befindet.

4. Verfahren nach einem der Ansprüche 1 bis 3, dadurch gekennzeichnet, daß die Reaktion in Anwesenheit eines mit dem Wasser mischbaren organischen Lösungsmittels erfolgt.

5. Verfahren nach Anspruch 4, dadurch gekennzeichnet, daß das verwendete Lösungsmittel Tetrahydrofuran ist.

6. Verfahren zur Herstellung des Dehydroxy-7,8 paramenthen-1, dadurch gekennzeichnet, daß man nach einem der Ansprüche 1 bis 5 verfährt, jedoch die Behandlung der organischen Phase mit einer Mineralsäure unterläßt.